# EUROPEAN PATENT APPLICATION

(11) **EP 4 796 102 A1**
(43) Date of publication of application: **26.08.2026**
(21) Application number: 26159822.1
(22) Date of filing: 20.02.2026
(51) Int. Cl.: A61K 8/34, A61K 8/41, A61K 8/73, A61Q 5/02, A61Q 5/12

(54) **HAIR CARE COMPOSITION FOR LEAVE-IN**

(30) Priority: 21.02.2025 EP 25159447
(71) Applicant: Kao Germany GmbH, 64297 Darmstadt (DE)
(72) Inventor: EBNER, Frank, 64297 Darmstadt (DE); Burgdorf, Kristin, 64297 Darmstadt (DE)
(74) Representative: Miller, Tobias

(57) **Abstract**

An aqueous cosmetic composition for keratin fibers, preferably human keratin fibers, more preferably human hair, comprising: a) one or more acetylated or non-acetylated glucomannan polymer(s), b) one or more linear or branched, saturated or unsaturated C₁₂-C₂₂ fatty alcohols, c) one or more conditioning agent(s).

## Description

### Field of the invention

The present invention relates to a leave-in cosmetic composition, a process for caring of keratin fibers, and a kit for fibers.

### Background of the invention

Beautifully soft, smooth, and healthy-looking hair gives many end-consumers a good feeling and increases self-confidence. Different product types are offered in the market with these benefits, like rinse-off emulsions and leave-in products. The leave-in products currently available on the market are mainly based on synthetic polymers to achieve the care and thickening effect, and on synthetic oils like silicones and mineral oil-based derivatives, making them less environmentally friendly. During the last years, environmentally friendly products have become more and more important for end consumers. Thus, there is a need to develop leave-in products based on natural polymers and natural oils.

### Summary of the invention

Therefore, the first object of the present invention is an aqueous cosmetic composition for keratin fibers, preferably human keratin fibers, more preferably human hair, comprising:
a) one or more acetylated or non-acetylated glucomannan polymer(s),
b) one or more linear or branched, saturated or unsaturated C₁₂-C₂₂ fatty alcohols,
c) one or more conditioning agent(s).

The second object of the present invention is a process for caring of keratin fibers, preferably human keratin fibers, more preferably for human hair, comprising the steps of:
i) optionally washing the hair with a cleansing composition and optionally drying the hair;
ii) applying the composition as defined above onto keratin fibers, especially wet or dry keratin fibers, and the keratin fibers are dried without rinsing off.

The third object of the present invention is a kit for keratin fibers comprising the composition as described above and a hair styling tool such as a brush or a comb or hair dryer.

### Detailed description of the invention

The inventors of the present invention surprisingly found that the composition, method and kit according to the independent claims showed better care performance with respect to smoothness, softness, and evenness to the tip.

### Aqueous composition for treating keratin fiber(s)

First aspect of the present invention is aqueous cosmetic composition for keratin fibers, preferably human keratin fibers, more preferably human hair, comprising:
a) one or more acetylated or non-acetylated glucomannan polymer(s),
b) one or more linear or branched, saturated or unsaturated C₁₂-C₂₂ fatty alcohols,
c) one or more conditioning agent(s).

### Compound(s) according to group a) - natural polymer(s)

The composition of the present invention comprises one or more acetylated or non-acetylated glucomannan polymer(s) as compound(s) according to group a). Preferably, these said glucomannan polymer(s) is/are one or more konjac glucomannan, acemannan, aloe glucomannan, dendrobium glucomannan, Bletilla glucomannan, and/or their mixtures, more preferably said compound(s) according to group a) are konjac glucomannans.

Preferably, the total concentration of compound(s) according to group a) in the composition of the present invention is in the range of 0.01% to 10% by weight, more preferably in the range of 0.1% to 3% by weight, still more preferably in the range of 0.5% to 2% by weight, still more preferably in the range of 1% to 2.5% by weight, calculated to the total weight of the composition.

### Compound(s) according to group b) - Fatty alcohol(s)

The composition of the present invention comprises one or more saturated or unsaturated C₁₂-C₂₂ fatty alcohols as compound(s) according to group b).

Preferably, the one or more saturated or unsaturated C₁₂-C₂₂ fatty alcohols is/are selected from one or more cetyl alcohol, stearyl alcohol, cetearyl alcohol, behenyl alcohol, arachidyl alcohol, myristyl alcohol, isocetly alcohol and oleyl alcohol, and mixtures thereof.

Preferably, the total concentration of compound(s) according to group b) in the composition of the present invention is in the range of 0.01% to 10% by weight, more preferably in the range of 0.1% to 5% by weight, still more preferably in the range of 0.5% to 3.5% by weight, still more preferably in the range of 1% to 2.5% by weight, calculated to the total weight of the composition.

### Compound(s) according to group c) - Conditioning agent(s)

The composition of the present invention comprises one or more conditioning agents as compound(s) according to group c). Preferably, said compounds is/are selected from esterquat and/or amidoamine surfactant(s), and/or their mixtures, more preferably the one or more esterquat surfactants are selected from dioleoylethyl hydroxyethylmonium, dicocoylethyl hydroxyethylmonium, and/or their salt(s), and preferably the one or more amidoamine surfactants are selected from brassicamidopropyl dimethylamine or behenamidoproyl dimethylmine, and/or their salt(s), and/or their mixtures, still more preferably the one or more compound(s) according to group c) is/are dicocoylethyl hydroxyethylmonium and/or its salt(s), preferably the methosulfate salt.

Preferably, the total concentration of compound(s) according to group c) in the composition of the present invention is in the range of 0.01% to 10% by weight, more preferably in the range of 0.1% to 5% by weight, still more preferably in the range of 0.25% to 3.5% by weight, still more preferably in the range of 0.3% to 1.5% by weight, calculated to the total weight of the composition.

### Aqueous composition

The term 'aqueous' within the meaning of the present invention denotes a composition that is based on water. Preferably the total concentration of water in the composition of the present invention is 85% by weight or more, preferably 90% by weight or more, more preferably 95% by weight or more, calculated to the total weight of the composition.

Preferably, the composition of the present invention has a pH in the range of 4.0 to 7.0, preferably in the range of 3.0 to 6.0, more preferably in the range of 3.5 to 5.0. The pH thereby may be measured with a calibrated glass electrode at 25°C in water.

It is preferred that the composition of the present invention is a hair care or styling composition, more preferably a leave-in hair care or styling composition.

### Anionic thickening polymers

The composition may further comprise one or more anionic thickening polymer(s), preferably said thickening polymers are sclerotium gum, Caesalpinia spinosa gum, hydrolyzed sclerotium gum, xanthan gum, dehydroxanthan gum, and or/their salt(s), and/or their mixtures.

Preferably, the total concentration of one or more anionic thickening polymer(s) is in the range of 0.1% to 5% by weight, more preferably in the range of 0.25% to 3.5% by weight, still more preferably in the range of 0.5% to 2% by weight, calculated to the total weight of the composition.

### Organic solvents

The composition may further comprise one or more organic solvents, preferably one or more C₂-C₄ alcohol(s), more preferably one or more C₂-C₄ mono-, di-, or trialcohols, still more preferably one or more ethanols, isopropanols, n-propanols, ethylene glycols, propylene glycols, glycerols, and/or their mixtures, still more preferably the composition further comprises propylene glycols, and preferably the total concentration of one or more organic solvents, still more preferably the total concentration of one or more C₂-C₄ alcohol(s) in the composition is in the range of 0.1% to 10% by weight, more preferably in the range of 0.25% to 7.5% by weight, still more preferably in the range of 0.5% to 5% by weight, calculated to the total weight of the composition.

### An aqueous hair care composition

The present invention is also directed to an aqueous hair care or styling composition having a pH in the range of 4.0 to 7.0 comprising:
a) one or more acetylated or non-acetylated glucomannan polymer(s), preferably one or more konjac mannans, at a total concentration of 0.1% to 3% by weight, calculated to the total weight of the aqueous composition,
b) one or more stearyl alcohols and/or cetyl alcohols, and/or mixtures, at a total concentration of 0.1% to 5% by weight, calculated to the total weight of the aqueous composition,
c) one or more esterquat surfactants selected from dioleoylethyl hydroxyethylmonium, dicocoylethyl hydroxyethylmonium, and/or their salt(s), at a total concentration of 0.1% to 5% by weight, calculated to the total weight of the aqueous composition,
wherein the total concentration of water in the aqueous composition is 85% by weight or more, calculated to the total weight of the aqueous composition.

### Process for treating keratin fiber(s)

Another aspect of the present invention is a process for caring of keratin fibers, preferably human keratin fibers, more preferably for human hair, comprising the steps of:
i) optionally washing the hair with a cleansing composition and optionally drying the hair;
ii) applying the composition as defined above onto keratin fibers, especially wet or dry keratin fibers, and the keratin fibers are dried without rinsing off.

Preferably, the keratin fibers in step ii) are styled with a hair styling tool such as a brush, comb, or hair dryer.

Preferably, in the step ii) the keratin fibers are styled with a hot tool at a temperature in the range of 40°C to 120°C, more preferably in the range of 50°C to 100°C.

### Kit for keratin fibers

Still another aspect of the present inventio is a kit for keratin fibers comprising the composition as defined above and a hair styling tool such as a brush or a comb or hair dryer.

The following examples are to illustrate the invention, but not to limit it.

### EXAMPLES

**Table 1 Inventive compositions and expert panel test results.**

| **Ingredients (INCI names)** | **[% by weight]** | | |
|---|---|---|---|
| | **Inventive Example 1** | **Comparative Example 1** | **Comparative Example 2** |
| Glucomannan | 0.7 | - | - |
| Sclerotium Gum | - | 0.7 | - |
| Dehydroxanthan Gum | - | - | 0.7 |
| Dicocoylethyl hydroxyethylmonium methosulfate | 0.5 | 0.5 | 0.5 |
| Propylene glycol | 1 | 1 | 1 |
| Phenoxyethanol | 0.7 | 0.7 | 0.7 |
| Cetearyl Alcohol | 1.5 | 1.5 | 1.5 |
| Perfume | 0.3 | 0.3 | 0.3 |
| pH | 4.0±0.5 | 4.0±0.5 | 4.0±0.5 |
| Water | Add to 100.00 | | |

| **Evaluation by the Expert Panel** | | | |
|---|---|---|---|
| Panel test criteria | Level of the attributes | | |
| Smoothness | 5 | 5 | 0 |
| Softness | 5 | 3 | 2 |
| Evenness to tip | 5 | 3 | 2 |

### Discussion of results

Best care results were achieved by the inventive composition including 0.7 wt.% glucomannan. Expert panel observed these results to be better compared to formulations including the same amount sclerotium gum or dehydroxanthan gum (s. Table 1).

### Methods

The effect of the inventions was tested by an expert panel. For this, damaged human hair tresses were used. The tresses were washed with a shampoo and subsequently towel-dried. 0.3 g of leave in product was applied and distributed evenly on the towel-dried hair tresses and blow-dried in the next by a blow dryer. The evaluation was conducted by expert panel of 10 participants. The expert panel was asked to select the hair tresses which displayed the highest degree of smoothness, softness, and evenness to the tip (s. Table 1 for results).

The effect was observed and confirmed by an expert panel. The *"in vivo*" sensory evaluation by hairdressers is a well-established method used since about 50 years in the test salon at Kao Germany GmbH. This type of sensory evaluation is an industry standard method for testing the properties of professional hair care products on human hair.

The standard sensory evaluation is executed on real hair models by well-trained hairdresser experts, which are specialized in specific product segments e.g., color, perm, styling, and care products to ensure the reliability and reproducibility of the tests. The evaluation of the formulations (s. Table 1) was conducted as monadic comparison on two different types of mannequin heads. Every head was analyzed by the hairdressers for each property (s. Table 1) on the scale between 1 and 5, wherein 1 stands for "bad", 2 stands for "not good", 3 stands for "medium", 4 stands for "good", and 5 stands for "very good" performance. Each mannequin head was evaluated by 10 stylists and a mean value was calculated (s. Table 1).

## Claims

1. An aqueous cosmetic composition for keratin fibers, preferably human keratin fibers, more preferably human hair, comprising:
a) one or more acetylated or non-acetylated glucomannan polymer(s),
b) one or more linear or branched, saturated or unsaturated C₁₂-C₂₂ fatty alcohols,
c) one or more conditioning agent(s).

2. The composition according to claim 1, **characterized in that** one or more compound(s) according to group a) is/are one or more konjac glucomannan, acemannan, aloe glucomannan, dendrobium glucomannan, Bletilla glucomannan, and/or their mixtures, preferably said compound(s) according to group a) are konjac glucomannans.

3. The composition according to claims 1 and/or 2, **characterized in that** the total concentration of compound(s) according to group a) is in the range of 0.01% to 10% by weight, preferably in the range of 0.1% to 3% by weight, more preferably in the range of 0.5% to 2% by weight, most preferably in the range of 1% to 2.5% by weight, calculated to the total weight of the composition.

4. The composition according to any of the preceding claims, **characterized in that** the one or more compound(s) according to group b) is/are one or more cetyl alcohol, stearyl alcohol, cetearyl alcohol, behenyl alcohol, arachidyl alcohol, myristyl alcohol, isocetly alcohol and oleyl alcohol, and mixtures thereof.

5. The composition according to any of the preceding claims, **characterized in that** the total concentration of compound(s) according to group b) is in the range of 0.01% to 10% by weight, preferably in the range of 0.1% to 5% by weight, more preferably in the range of 0.5% to 3.5% by weight, most preferably in the range of 1% to 2.5% by weight, calculated to the total weight of the composition.

6. The composition according to any of the preceding claims, **characterized in that** the one or more compound(s) according to group c) is/are one or more conditioning agents selected from esterquat and/or amidoamine surfactant(s), and/or their mixtures, preferably the one or more esterquat surfactants are selected from dioleoylethyl hydroxyethylmonium, dicocoylethyl hydroxyethylmonium, and/or their salt(s), and preferably the one or more amidoamine surfactants are selected from brassicamidopropyl dimethylamine or behenamidoproyl dimethylmine, and/or their salt(s), and/or their mixtures, still more preferably one or more compound(s) according to group c) is/are dicocoylethyl hydroxyethylmonium, and/or its salts, preferably the methosulfate salt.

7. The composition according to any of the preceding claims, **characterized in that** the total concentration of compound(s) according to group c) is in the range of 0.01% to 10% by weight, preferably in the range of 0.1% to 5% by weight, more preferably in the range of 0.25% to 3.5% by weight, most preferably in the range 0.3% to 1.5% by weight, calculated to the total weight of the composition.

8. The composition according to any of the preceding claims, **characterized in that** the composition further comprises one or more anionic thickening polymer(s), preferably said thickening polymers are sclerotium gum, Caesalpinia spinosa gum, hydrolyzed sclerotium gum, xanthan gum, dehydroxanthan gum, and or/their salt(s), and/or their mixtures, and preferably the total concentration of one or more anionic thickening polymer(s) is in the range of 0.1% to 5% by weight, more preferably in the range of 0.25% to 3.5% by weight, still more preferably in the range of 0.5% to 2% by weight, calculated to the total weight of the composition.

9. The composition according to any of the preceding claims, **characterized in that** the total concentration of water in the composition is 85% by weight or more, preferably 90% by weight or more, more preferably 95% by weight or more, calculated to the total weight of the composition.

10. The composition according to any of the preceding claims, **characterized in that** the composition has a pH in the range of 4.0 to 7.0, preferably in the range of 3.0 to 6.0, more preferably in the range of 3.5 to 5.0.

11. The composition according to any of the preceding claims, **characterized in that** the composition further comprises one or more organic solvents, preferably one or more C₂-C₄ alcohol(s), more preferably one or more C₂-C₄ mono-, di-, or trialcohols, still more preferably one or more ethanols, isopropanols, n-propanols, ethylene glycols, propylene glycols, glycerols, and/or their mixtures, still more preferably the composition further comprises propylene glycols, and preferably the total concentration of one or more organic solvents, still more preferably the total concentration of one or more C₂-C₄ alcohol(s) in the composition, is in the range of 0.1% to 10% by weight, more preferably in the range of 0.25% to 7.5% by weight, still more preferably in the range of 0.5% to 5% by weight, calculated to the total weight of the composition.

12. An aqueous hair care composition according to any of the preceding claims having a pH in the range of 4.0 to 7.0 comprising:
a) one or more acetylated or non-acetylated glucomannan polymer(s), preferably one or more konjac mannans, at a total concentration of 0.1% to 3% by weight, calculated to the total weight of the aqueous composition,
b) one or more stearyl alcohols and/or cetyl alcohols, and/or mixtures, at a total concentration of 0.1% to 5% by weight, calculated to the total weight of the aqueous composition,
c) one or more esterquat surfactants selected from dioleoylethyl hydroxyethylmonium, dicocoylethyl hydroxyethylmonium, and/or their salt(s), at a total concentration of 0.1% to 5% by weight, calculated to the total weight of the aqueous composition,
wherein the total concentration of water in the aqueous composition is 85% by weight or more, calculated to the total weight of the aqueous composition.

13. Process for caring of keratin fibers, preferably human keratin fibers, more preferably for human hair, comprising the steps of:
i) optionally washing the hair with a cleansing composition and optionally drying the keratin fibers;
ii) applying the composition according to any of the claims 1 to 12 onto keratin fibers, especially wet or dry keratin fibers, then the keratin fibers are dried without rinsing off.

14. Kit for keratin fibers comprising the composition according to any of the claims 1 to 12 and a hair styling tool such as a brush or a comb or hair dryer.
